# EUROPEAN PATENT APPLICATION

(11) **EP 2 106 801 A1**
(43) Date of publication of application: **07.10.2009**
(21) Application number: 08154101.3
(22) Date of filing: 04.04.2008
(51) Int. Cl.: A61K 38/20, C07K 14/54, A61P 29/00, A61P 37/00, A61P 1/00, A61K 39/395

(54) **IL-25 for use in the treatment of inflammatory diseases**

(71) Applicant: Giuliani International Limited, 20129 Milano (IT)
(72) Inventor: Monteleone, Giovanni, I-00133, Rome (IT)
(74) Representative: Gates, Marie Christina Esther

(57) **Abstract**

The current invention relates to the regulation of cell cytokine response, in particular the cytokine response of CD 14⁺ cells. The cytokines are involved in the Th1 response and / or the Th-17 response. The invention further relates to the use of cytokines in the preparation of a medicament for the treatment of inflammatory disease in which CD14+ cells play a part. The disease is an inflammatory disease including, but not limited to inflammatory bowel disease (IBD), Crohn's disease (CD), ulcerative colitis, coronary artery disease, sepsis, rheumatoid arthritis, immune-complex induced and proliferative glomerulonephritis, tubulointerstitial nephritis, vasculitis, viral and autoimmune hepatitis, autoantibody-mediated disease, psoriasis, neoplasia and other CD 14⁺ cell related diseases.

## Description

### Field of the Invention

The current invention relates to the regulation of cell cytokine response, in particular CD14⁺ cells. The invention further relates to the use of cytokines in the preparation of a medicament for the treatment of inflammatory diseases including, but not limited to inflammatory bowel disease (IBD), Crohn's disease (CD), ulcerative colitis, coronary artery disease, sepsis, arthritis rheumatoid, immune-complex induced and proliferative glomerulonephritis, tubulointerstitial nephritis, vasculitis, viral and autoimmune hepatitis, autoantibody-mediated diseases, psoriasis, neoplasia and other CD14⁺ cell related diseases.

### Background to the Invention

Interleukin (IL)-25, also known as IL-17E, is a member of the structurally related IL-17 cytokine family. Its in vivo and in vitro biological functions are markedly different from other IL-17 family members however. IL-17 is expressed by activated CD4⁺ T-cells and mediates the induction of pro-inflammatory cytokines such as IL-1β and IL-6 and TNFα. IL-25 on the other-hand is expressed in activated Th2 T-cells and in non-T cells. Transgenic expression of IL-25 and systemic administration of the recombinant cytokine in mice has been shown to induce a Th-2 cell-associated mucosal inflammatory pathologies. A Th-2 response is characterized by epithelial cell hyperplasia, increased mucous production, and recruitment of inflammatory cells into inflamed tissues with elevated expression of IL-4 and IL-5 and IL-13. In contrast, studies in murine models of autoimmunity have shown that IL-25 can negatively regulate the development and/or amplification of Th17-mediated pathology. Therefore, it has been shown that IL-25 can either promote or inhibit specific inflammatory responses. Literature also suggests that IL-25 has a role in promoting allergic responses. However, the active role IL-25 plays in the regulation of immunity and infection induced inflammation in vivo remains largely unknown.

The biological functions of IL-25 are mediated by a receptor called IL-25R. IL-25R was first identified as an IL-17B receptor (IL-17BR), also called IL-17 receptor homolog 1 and EVI27. IL-25R is a single pass transmembrane receptor that is expressed in the kidneys, liver, intestine and on the surface of human basophils and dendritic cells that have been activated in the presence of Th2-associated stimuli.

Inflammatory bowel disease (IBD) is a group of inflammatory conditions of the large intestine and / or small intestine. IBD represent chronic tissue-destructive diseases, the etiology of which remains unknown. The main forms of IBD are Crohn's disease (CD) and ulcerative colitis. IBD is characterised by an infiltration of CD14+ cells to the intestinal mucosa. Evidence suggests that CD14+ cells play a role in the tissue-damaging immune response, due to their ability to produce huge amounts of inflammatory cytokines. In relation to Crohn's disease, mucosal CD14+ cells synthesise high levels of Interleukin (IL)-12 and IL-23. These cytokines are involved in the control of the Th1 and Th17 cell response, respectively. These responses in turn, result in the production of IFN-gamma and IL-17 and IL-22, respectively. In CD and ulcerative colitis, mucosal CD14+ cells are also a source of tissue necrosis factor (TNF)-α, IL-1β, IL-6 and IL-8. The molecular mechanisms underlying the enhanced production of inflammatory cytokines by CD14 cells in IBD is not fully understood.

In general, steroids, immunosuppressors, and anti-TNF-alpha antibodies are drugs widely used in CD14+ cell-related pathologies (e.g. psoriasis, vasculitis, rheumatoid arthritis). The therapeutic intervention for patients with Crohn's disease and patients with ulcerative colitis is markedly influenced by the type and severity of the clinical symptoms, the type and the extension of the lesions, as well as by the presence of any complications. In general, salicylazosulfapyridine and 5-aminosalicylic acid are drugs of proven efficacy in the management of patients with mild IBD and for prolonging remission of the disease. Moderate-severe IBD cases require the use of cortico-steroids and clinical remission is obtainable in two thirds of patients receiving such a treatment. However, it is reported that approximately 25% of patients undergoing such treatment experience steroid-dependence. Moreover, chronic administration of steroids is associated with a high risk of adverse side effects. Therefore, these subgroups of patients who do not respond well or cannot tolerate steroid treatment are candidates for treatment with immunosuppressors such as azathioprine, 6-mercaptopurine, cyclosporine, and methotrexate or biological drugs for example anti-TNF-α antibodies. In general however, the use of these compounds is also associated with enhanced risk of severe side effects and approximately 50% of patients are resistant to such a treatment.

A previous study by Fort MM et al, disclosed the presence of IL-25 RNA in the gastrointestinal tract of mice. However, the association of IL-25 with inflammatory bowel disease in mice or humans has never been proven. WO2006088833 recently disclosed polypeptides related to the IL-17 signalling pathway. In particular, it reported the potential use for signal antagonists of the IL-17F signalling pathway in methods of treating diseases related to IL-17F signalling, such as inflammatory diseases. EP1863520 disclosed the use of IL-25 to inhibit tumour growth. The anti-tumour effect of IL-25 described in EP1863520 is attributed to increased infiltration into the tumour site. WO200409684 disclosed a number of peptides including IL-25, linked to a carrier protein to form a vaccine compound. The described vaccine functions to reduce allergic responses, induced by over-expressed cytokines by way of the induction of auto-antibodies that bind to cytokines and thus prevent the allergic response.

There is therefore a need, to provide a more effective mode of treatment for inflammatory diseases with reduced risk of the severe side effects associated with the current methods of treatment. In contrast to the teachings of the above-mentioned patents and scientific literature to date, the current invention provides the use of IL-25 as an alternative medicament for the treatment of inflammatory diseases.

### Object of the Invention

It is an object of the current invention to provide a means of regulating the cytokine production of cells, in particular CD14+ cells. It is a further object of the invention to provide an alternative medicament for the treatment of inflammatory disease, in which CD14+ plays a role. The disease can be inflammatory bowel disease Crohn's disease (CD), ulcerative colitis, coronary artery disease, sepsis, arthritis rheumatoid, immune-complex induced and proliferative glomerulonephritis, tubulointerstitial nephritis, vasculitis, viral and autoimmune hepatitis, autoantibody-mediated diseases, psoriasis and neoplasia and other CD 14+ cell related diseases.

### Summary of the Invention

The current invention provides a means of regulating the cytokine production of cells comprising interleukin (IL)-25, a derivative of or a fragment of IL-25. These cells can be CD14+ cells. The cytokines are involved in the TH1 response and /or TH-17 response. The cytokines can be IL-6 and IL-8, TNF-α, IL12, IL-23, IL-22, IL-21 IFN-gamma, IL-17 and IL-1β. The regulation of the cytokine response of CD14+ cells by IL-25 may be dependent on SOCS-3.

The invention further provides for the use of IL-25 a derivative of or a fragment of IL-25, in the preparation of a medicament for the treatment of disease. The disease may be an inflammatory disease in which CD14+ cells play a part. The disease can be but is not limited to inflammatory bowel disease (IBD) such as Crohn's disease (CD) or ulcerative colitis, coronary artery disease, sepsis, arthritis rheumatoid, immune-complex induced and proliferative glomerulonephritis, tubulointerstitial nephritis, vasculitis, viral and autoimmune hepatitis, autoantibody-mediated diseases, psoriasis and neoplasia and other CD 14+ cell related diseases.

A further embodiment of the present invention provides for a pharmaceutical composition containing a pharmaceutically active amount of an interleukin for the treatment of disease. The interleukin may be interleukin-25, a derivative of or a fragment of IL-25.

The invention further provides for a method of down regulating the inflammatory cytokine response of CD14+cells, comprising administering of interleukin-25, a derivative or a fragment thereof.

A further embodiment of the current invention provides for an antibody directed at IL-25R in the preparation of a medicament for the treatment of disease. An antibody to IL-25R, will mimic the **mode** of action of IL-25.

The present invention will now be described with reference to the following figures.

### Brief Description of the Figures

**Figure 1****.** Real-time PCR expression of the IL-25 receptor (IL-25R) in human blood CD 14+ cells.
**Figure 2****.** Inhibition of inflammatory cytokines induced by LPS and PGN in human blood CD14+ cells by IL-25.
**Figure 3****.** TLR-2 and TLR-4 levels in blood CD14+ cells cultured in the presence or absence of PGN (A) or LPS (B).
**Figure 5** CD80, CD86, and HLA-DRII levels in blood CD14+ cells cultured in the presence or absence of LPS, PGN, or CpG.
**Figure 6****.** Phagocytic activity of CD14+ cells in response to IL-25 stimulation
**Figure 7****.** SOCS3 levels in response to IL-25 stimulation in blood CD14+ cells induced by LPS and PGN
**Figure 8****.** IL-25 mediated regulation of TNF-alpha and IL-6 in the absence of SOCS-3.
**Figure 9****.** IL-10 expression levels in CD14+ cells stimulated with IL-25.
**Figure 10****.** Real-time PCR data showing the expression of IL-25R in intestinal CD14+ cells and are functionally capable of responding to IL-25.
**Figure 11****.** IL-25 expression is down regulated in the inflamed colon of patients with IBD.
**Figure 12****.** IL-25 expression is down regulated in experimental models of IBD.
**Figure 13****.**
   a) IL-25 prevents the induction of PGN-mediated colitis.
   b) Histological evaluation of PGN-cofitis in mice treated or untreated with IL-25
   c) Expression ofIL-12/p70 in mice with PGN-colitis treated or untreated with IL-25
**Figure 14****.**
   a) Percentage of mortality in control mice and mice with PGN-colitis treated or untreated with IL-25
   b) Body weight changes of control mice and mice with PGN-colitis treated or untreated with IL-25
   c) Macroscopic pictures of colon samples of control mice and mice with PGN-colitis treated or untreated with IL-25.
**Figure 15****.**
   a) Percentage of mortality in control (ETOH) mice and colitic mice (TNBS-induced) left untreated or treated with IL-25.
   b) Representative macroscopic pictures of colons in control (ETOH) and colitic mice either left untreated or treated with IL-25.
   c) Macroscopic pictures of colon samples of control mice and mice with TNBS-colitis treated or untreated with IL-25.
   d) Expression ofIL-12/p70 in mice with TNBS-colitis treated or untreated with IL-25
   e) Expression of IFN-γ in mice with TNBS-colitis treated or untreated with IL-25

### Detailed Description of the Invention

### Materials and Methods

### Cell Culture

Human peripheral blood mononuclear cells (PBMC) were isolated from enriched buffy coats of healthy volunteer donors and patients with IBD. PBMC were used as a source for the isolation and purification of CD 14+ and CD3+ T cells, carried out using CD14 or CD3 magnetic beads (Miltenyi Biotec, Bologna, Italy). Cell purity was routinely evaluated by flow cytometry and ranged between 96% and 98%. All reagents were from Sigma-Aldrich (Milan, Italy) unless specified. RNA was extracted from an aliquot of PBMCs and assessed for IL-25R expression by flow-cytometry. The remaining cells were resuspended in RPMI 1640 medium, supplemented with 10% inactivated fetal bovine serum (FBS), penicillin (100 U/mL), and streptomycin (100 µg/mL) (Life Technologies-GibcoCRL, Milan, Italy), and seeded in 48-well culture dishes (1x10⁶ cells/well). CD14+ cells were pre-incubated with IL-25 (50 ng/ml, R&D Systems, Inc. Minneapolis, MN, USA) for 30 minutes and then stimulated with LPS (100 ng/ml) or PGN (10µg/ml) or CpG (5µg/ml) or TNF-α (R&D Systems), or IFN-γ (50 ng/ml, Peprotech EC LTD, London, UK) for 30 minutes to 48 hours. RNA was then extracted from the cells for cell-surface protein analysis.

### RNA extraction and cDNA preparation

RNA was extracted from TRIzol reagent according to the manufacturer's instructions (Invitrogen, Milan, Italy). A constant amount of RNA (500 ng/sample) was retro-transcribed into complementary DNA (cDNA).

### Real-time PCR

1 µl of cDNA from each sample was amplified using the following conditions outlined in Table 1.

**Table 1 Real-Time PCR conditions**

| **Step** | **Time** | **Temperature** |
|---|---|---|
| **Denaturation** | 1 min | 95°C |
| **Annealing** | 30 sec | 58°C (human IL-23/p19, human IL-8) |
| | | 60°C (human IL-6) |
| | | 62°C (human TNF-α, human IL-17A, human IL-25R membrane-bound isoform, murine/β-actin) |
| **Extension** | 30 sec | 72°C |

Primers sequences used in the current invention are as listed in Table 2

**Table 2: Primers sequences**

| **Primer Name** | **Primer Sequence** |
|---|---|
| Human IL-23/p19 FWD | 5'-GGGACACATGGATCTAAGAG-3' |
| Human IL-23/p19 REV | 5'-GCAAGCAGAACTGACTGTTG-3' |
| Human IL-8 FWD | 5'-AGGAACCATCTCACTGTGTG-3' |
| Human IL-8 REV | 5'-CCACTCTCAATCACTCTCAG-3' |
| Human IL-6 FWD | 5'-CCACTCACCTCTTCAGAACG-3' |
| Human IL-6 REV | 5'-GCCTCTTTGCTGCTTTCACAC-3' |
| Human TNF-α FWD | 5'-AGGCGGTGCTTGTTCCTCAG-3' |
| Human TNF-α REV | 5'-GGCTACAGGCTTGTCACTCG-3' |
| Human IL-17A FWD | 5'-ACTACAACCGATCCACCTCAC-3' |
| Human IL-17A REV | 5'-ACTTTGCCTCCCAGATCACAG-3' |
| Human IL-25R membrane-bound isoform FWD | 5'-CCTCCGAGTAGAACCTGTTAC-3' |
| Human IL-25R membrane-bound isoform REV | 5'-AGTTGCTTTTGCCCGTCACAC-3', |
| Human IL-25 soluble form FWD | 5'-AGGTGGGGACACAGGAGGTC-3' |
| Human IL-25 soluble form REV | 5'-CTGGAGGACACAGGGGTGGA-3'. |
| β-actin FWD | 5'AAGATGACCCAGATCATGTTTGAGACC-3' |
| β-actin REV | 5'-AGCCAGTCCAGACGCAGGAT-3 |

The expression levels of murine and human IL-25, human IL-23/p40, human IL-12/p35, human SOCS-1 and SOCS-3 were evaluated using a commercially available TaqMan probe (Applied Biosystems, Foster City, CA, USA). β-action was used as an internal control. Real-time PCR was performed using the IQ SYBR Green Supermix (Bio-Rad Laboratories, Milan, Italy).

### Flow-Cytometry

The level of IL-25R was evaluated in total PBMC and in purified blood and intestinal CD3+ T and CD14+ cells using the following monoclonal anti-human antibodies:
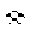 CD56 FITC (1:10 final dilution, Diaclone Research, Milan, Italy)
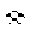 CD3 PerCP (1:50, final dilution, Becton Dickinson, Milan, Italy)
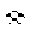 CD14 FITC (1:50 final dilution, Immunotools, Friesoythe; Germany)
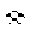 CD19 (1:50 final dilution, Immunotools)
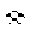 Isotype control IgG (Becton Dickinson, Milan, Italy)
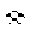 IL-25R PE (1:10 final dilution, R&D Systems)

To evaluate cell activation markers, CD 14+ cells were targeted by the following antibodies:
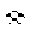 anti-human TLR4 PE or TLR2 PE (both at 1:5 final dilution, eBioscience, San Diego, CA, USA)
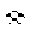 anti-human HLD-DRII PE or anti-human CD86 FITC (both at 1:50 final dilution, Becton Dickinson)
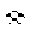 anti-human CD80 APC (1:50 final dilution, Immunotools)

Cells were then incubated at 4°C for 30 minutes. Cells were washed, resuspended in PBS and analyzed by flow-cytometry. In order to determine the effect of IL-25 on the phagocytic properties of CD14+ cells, CD14+ cells were seeded in a 6-well dishes (1x10⁶ cells/ml) and pre-incubated with IL-25 (50 ng/ml) for 30 minutes and then stimulated with LPS (100 ng/ml) or PGN (10µg/ml) in presence of orange fluorescent latex beads (5x10⁶ beads/ml) for 6 hours at 37°C. Cells were then washed, resuspended in PBS and analyzed by flow-cytometry.

### SOC-3

To examine the role of SOCS-3 in the IL-25-mediated negative regulation of CD14+ cell cytokine response, CD14+ cells were cultured with and without human SOCS-3 or control siRNA according to the manufacturer instructions (Santa Cruz Biotechnology, Santa Cruz, CA). After 48 hours, cells were washed and cultured with or without IL-25 for 30 minutes and then stimulated with LPS or PGN for further 6 hours.

### Tissue Samples

Colonic biopsies were taken during endoscopy from inflamed areas of 9 patients with CD. In 5 out of 9 CD patients, the primary site of involvement was the terminal ileum and colon, while the remaining patients had a colonic disease. Two CD patients were receiving treatment with mesalazine, 5 patients were receiving steroids and mesalazine and the remaining patients were taking antibiotics. Additional mucosal samples were taken from intestinal resection specimens of 4 patients undergoing surgery for a chronic active disease poorly responsive to medical treatment. At the time of surgery, 2 patients were receiving corticosteroids and 2 patients were receiving antibiotics and corticosteroids.

Control samples include 12 patients with ulcerative colitis (UC): 6 with a left-sided colitis and 6 with an extensive disease. Two UC patients were on oral and topical mesalazine and the remaining patients were receiving steroids and topical mesalazine. "Normal" control samples include colonic mucosal samples taken from macroscopically and microscopically unaffected areas of 9 subjects undergoing colonoscopy for colorectal cancer screening, and 8 subjects undergoing colon resection for colon cancer.

### Lamina propria mononuclear cell isolation and cell culture

Lamina propria mononuclear cells (LPMC) were prepared by using the DTT-EDTA-collagenase procedure and used as a source for the purification CD 14+ and CD3+ T-cells by positive MACS separation. The resulting cell preparations contained >95% CD14+ and >95% CD3+ T cells, as assessed by flow cytometry. RNA was extracted from an aliquot of cells and assessed for the expression of IL-25R by real-time PCR and flow-cytometry. The remaining cells were resuspended in RPMI 1640 medium, supplemented with 10% inactivated FBS, penicillin (100 U/mL), and streptomycin (100 g/mL), and seeded in 48-well culture dishes (1x10⁶ cells/well). CD14+ cells were cultured with and without IL-25 (50 ng/ml) for 30 minutes and then stimulated with LPS. CD3+ T cells were cultured with or without IL-25 (50 ng/ml) for 30 minutes and then stimulated with activating anti-CD3+anti-CD28 antibodies. After 6 hours, cells were harvested and used for RNA extraction.

### Induction of trinitrobenzene sulfonic acid (TNBS) and Oxazolone colitis

Studies of hapten-induced colitis were performed in 5- to 6-week-old male SJL mice (Harlan Laboratories, S. Pietro al Natisone, UD, Italy), which were maintained in the animal facility at the Istituto Superiore di Sanità (Rome, Italy). The experiments were performed within 7 days of the arrival of the animals. For induction of colitis, 2.5 mg of TNBS or 6 mg of oxazolone in 50% ethanol was administered to lightly anesthetized mice through a 3.5F catheter inserted into the rectum. The catheter tip was inserted 4 cm proximal to the anal verge, and 150 µl of fluid was slowly instilled into the colon, after which the mouse was held in a vertical position for 30 seconds. Controls consisted of mice treated with 150 µL of 50% ethanol and untreated naive mice. Weight changes were recorded daily to assess the induction of colitis, and tissues were collected for histologic study and protein analysis. For histologic analysis, tissues were fixed in 10% neutral buffered formalin solution, embedded in paraffin, cut into tissue sections, and stained with H&E. For TNBS-induced colitis, stained sections were examined for evidence of colitis and assigned a colitis score by considering the presence of acute and chronic inflammatory infiltrates, elongation and/or distortion of crypts, frank ulceration, and thickening of the bowel wall. Additionally, colonic tissues taken from TNBS-induced colitis was used for RNA and protein extraction.
For oxazolone-induced colitis, stained sections were examined and assigned a colitis score by examining the slide for the presence of hypervascularization, mononuclear cells, epithelial hyperplasia, epithelial injury, and granulocytes. Colonic tissues taken from Oxazolone-induced colitis were used for protein extraction.

### Induction of Peptidoglycan (PGN) colitis and IL-25 administration

PGN colitis was induced in Balb/c mice by intrarectal administration of 100 µl of 50% ethanol followed 8 hours later by soluble PGN (1mg/mouse) again per rectum. Controls included mice that were given intrarectal administration with 50% ethanol but not PGN. Recombinant mouse IL-25 (10µg/mouse) was injected intra-peritoneally 1 hour before the administration of PGN, in the preventive model, whereas the cytokines was injected 1 day after the administration of PGN, in the therapeutic model. Weight changes were recorded daily to assess the induction of colitis, and tissues were collected for histologic study, protein analysis, and isolation of splenocytes and colonic LPMC. For histologic analysis, tissues were fixed in 10% neutral buffered formalin solution, embedded in paraffin, cut into tissue sections, and stained with H&E. In order to evaluate the effect of treatment with IL-25 in PGN-mediated colitis, IL-25 was administered (10µg/mouse) to Balb/c mice the day after the induction of colitis. Body weight changes were recorded daily in control and colitic mice either treated or untreated with IL-25.

### Protein extraction, Western blotting and IL-12 ELISA

IL-25 protein was analyzed using total proteins extracted from biopsies of 8 CD patients, 8 UC patients and 8 normal controls. Additionally, IL-25 was evaluated in colonic samples of 6 TNBS-induced colitis mice, 3 Oxazolone-induced colitis mice and 6 controls. Samples were lysed for 60 min on ice in buffer containing 10 mM Hepes (pH 7.9), 10 mM KCl, 0.1 mM EDTA, 0.2 mM EGTA, and 0,5% Nonidet P40, supplemented with 1 mM DTT, 10 µg/ml aprotinin, 10µ g/ml leupeptin, 1 mM phenylmethanesulphonyl fluoride (PMSF), 1 mM Na₃VO₄, and 1 mM NaF (all reagents were from Sigma-Aldrich). Lysates were then clarified by centrifugation at 4 °C for 30 minutes at 12,000 x g. Equal amounts of total proteins were fractionated on SDS-polyacrylamide gels. The membranes were blocked with Tris-buffered saline containing 0.05% Tween 20 and 5% nonfat dry milk and then incubated with a mouse anti-human IL-25 (1 µg/ml) or with a rat antimouse IL-25 (1 µg/ml, both from R&D Systems) followed by a horseradish peroxidase-conjugated secondary antibodies (1:20000 final dilution, Dako, Milan, Italy). The reaction was detected with a chemiluminescence kit (West DURA; Pierce, Rockford, IL). Computer-assisted scanning densitometry (Total lab, AB.EL S.r.l., Rome, Italy) was used to analyze the intensity of the immunoreactive bands.
Total proteins extracted from mice with PGN-colitis and controls either left untreated or treated with IL-25 were used to analyze IL-12/p70 by a commercially available ELISA kit (R&D Systems) according to the manufacturer's instructions.

### RESULTS

### IL-25R expression in CD14+ cells

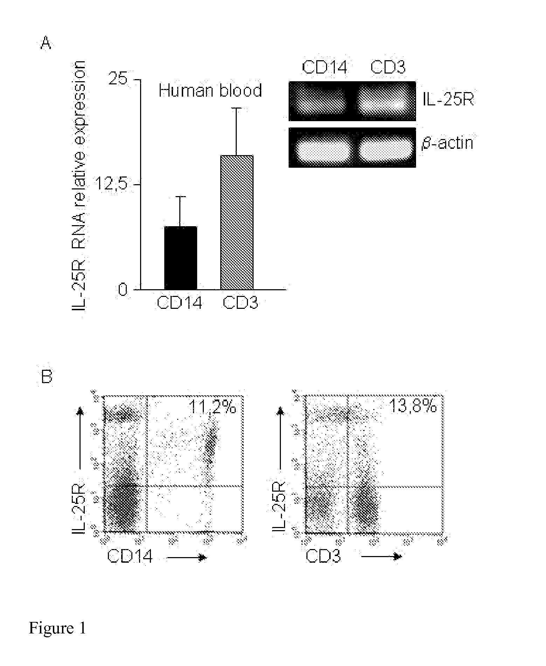

The expression of IL-25R was analysed in blood CD14+ and CD3+ cells by Real-Time PCR. The transcripts for both the membrane-bound and soluble IL-25R isoforms were constitutively expressed in blood CD14+ cells (Fig. 1A). Flow-cytometry analysis confirmed that CD14+ cells express the cell surface-associated IL-25R.

### Response of CD14+ to stimuli

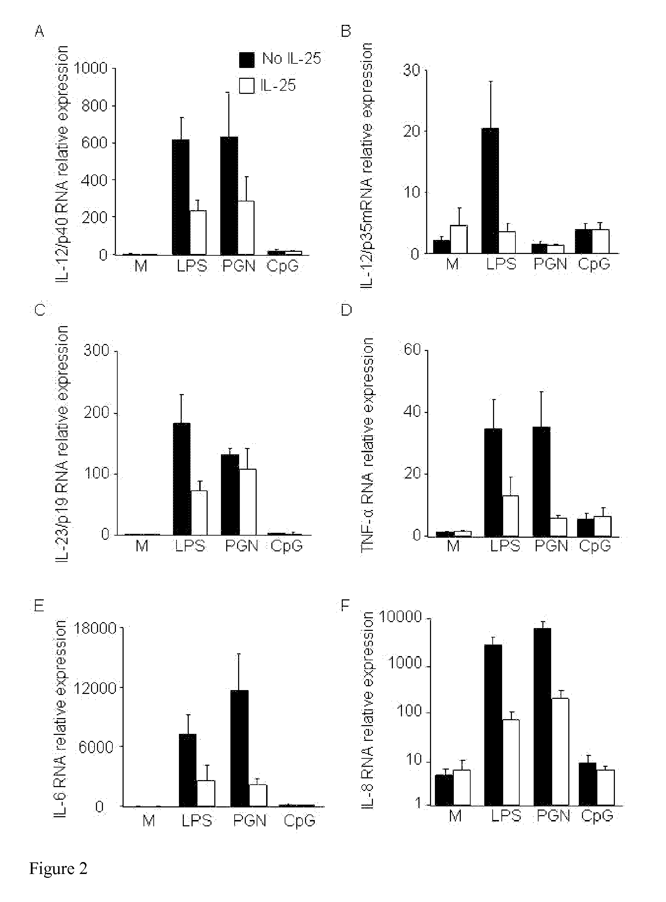

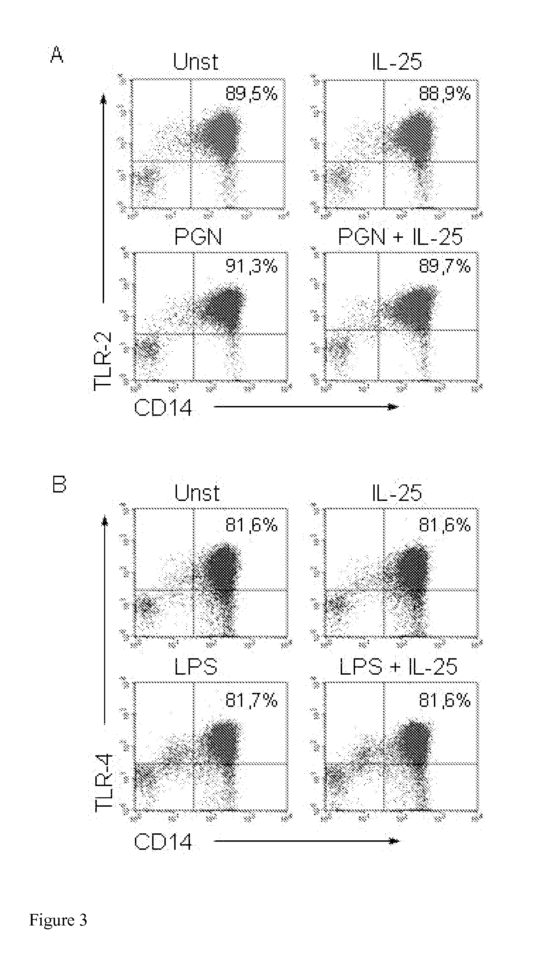

CD14+ cells were cultured with or without IL-25, in the presence or absence of various bacterial stimuli. As shown in figure 2, IL-25 markedly inhibited the RNA expression of several inflammatory cytokines i.e. IL-12, IL-23, IL-6, IL-8, and TNF-α induced by the presence of lipopolysaccharide (LPS) and peptidoglycan (PGN). CD14+ cells were less sensitive to stimulation by CpG motifs.
The induction of IL-23/p19 and TNF-alpha by CpG was not modified by IL-25. One explanation for the IL-25-mediated reduced expression of LPS/PGN-stimulated inflammatory cytokines is the down-regulation of cell-surface receptors that mediate LPS/PGN signals. The expression of both toll-like receptor (TLR)-2 and TLR-4, at early (i.e. 6 hours, Fig. 3) and late (i.e. 48 hours, not shown) time points in cells either left unstimulated or stimulated with LPS or PGN was not modified by the presence of IL-25.

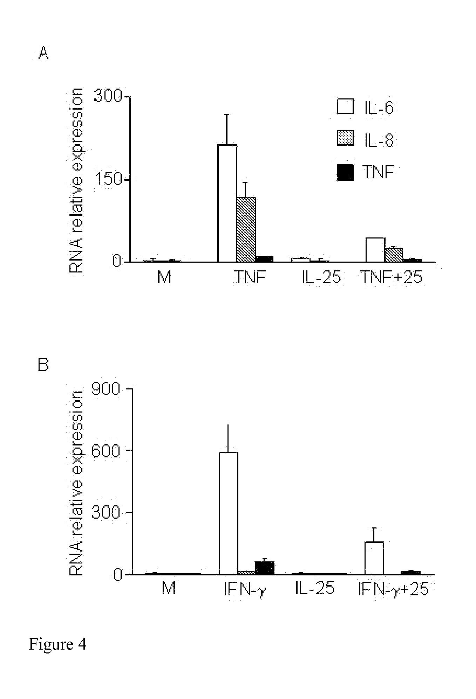

To assess whether IL-25 renders CD 14+ cells resistant to additional inflammatory stimuli, CD14+ cells were pre-incubated with IL-25 for 30 minutes and then stimulated with IFN-gamma or TNF-alpha. As expected, the expression of IL-6 and IL-8 RNA transcripts was enhanced by TNF-alpha. Such an induction was significantly inhibited by IL-25 (Fig. 4A). Additionally, IL-25 reduced the expression of IL-6 and TNF-alpha transcripts induced by IFN-gamma (Fig. 4B).

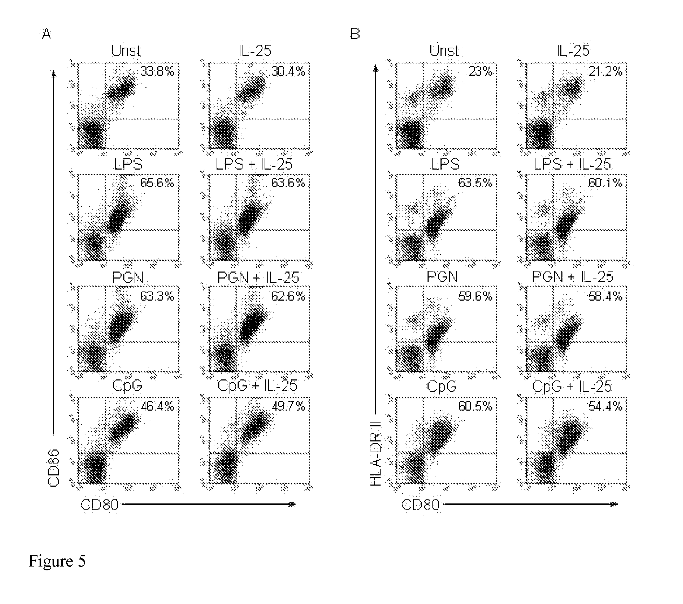

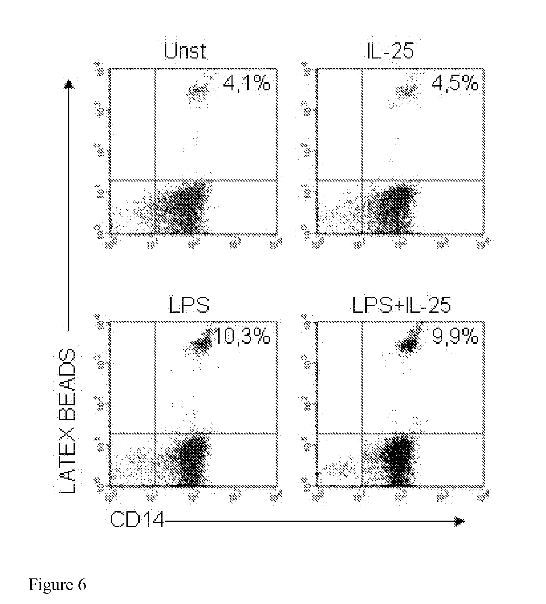

To exclude the possibility that IL-25 causes a global hyporesponsiveness of CD 14+ cells, the expression levels of CD80, CD86, and HLA-DRII was investigated. CD14+ cells were treated with IL-25 or were left untreated. IL-25 did not reduce the fraction of CD80, CD86, and HLA-DRII induced by 6 and 48-hour stimulation with bacterial stimuli (Fig. 5 and not shown). Moreover, the ability of both unstimulated and LPS-stimulated CD14+ cells to phagocyte latex beads was not affected by IL-25 (Fig. 6). Overall these data indicate that IL-25 selectively inhibits the production of inflammatory cytokines rather than making CD 14+ cells resistant against bacterial/cytokine stimuli.

### Knockdown of SOC-3 by siRNA

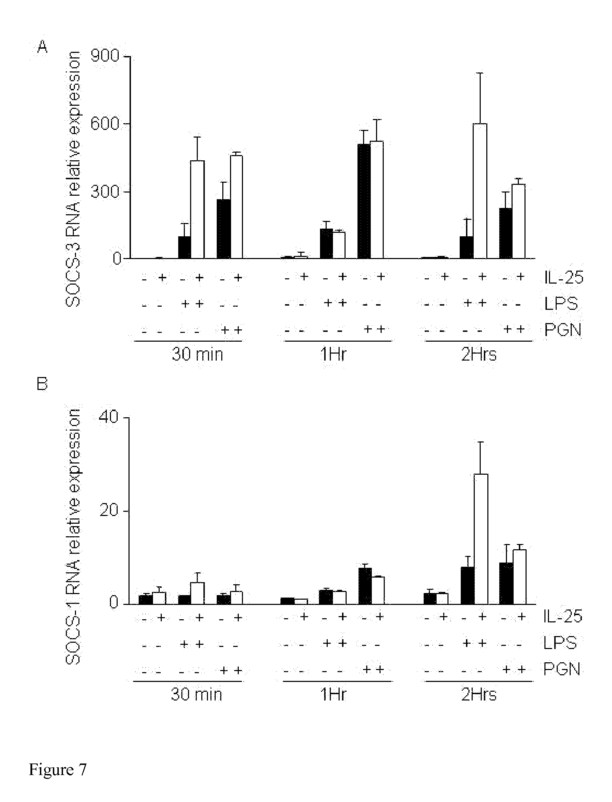

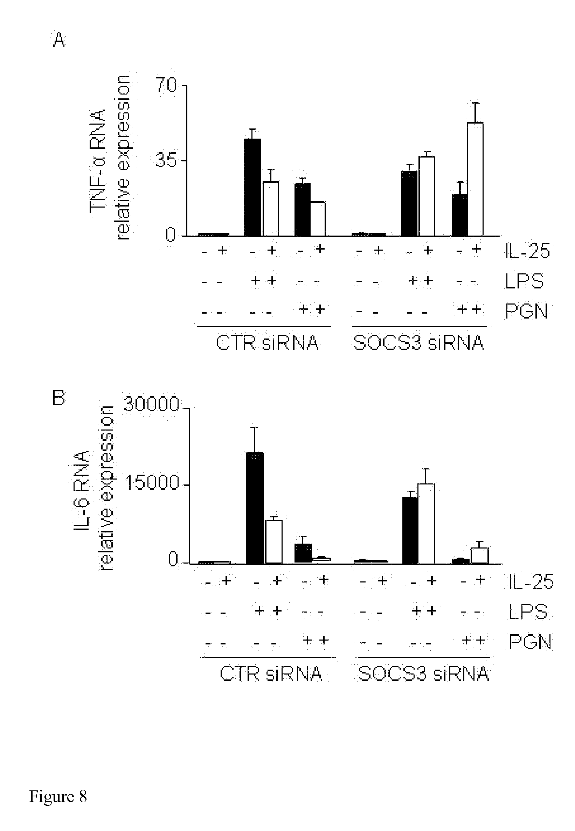

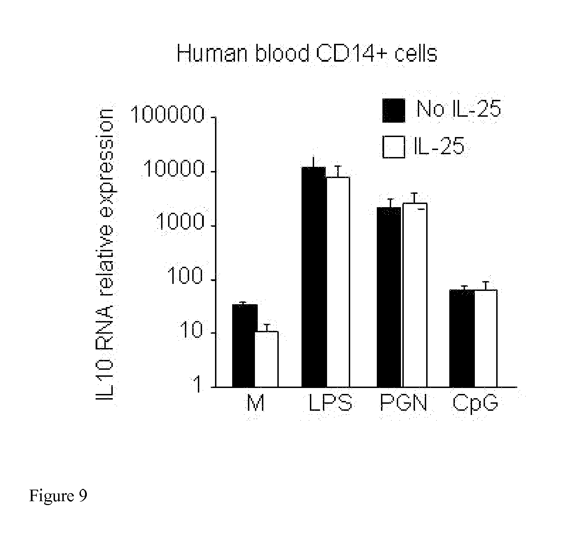

The effect of IL-25 on CD14+ cell response was examined for dependency on the induction and/ or activity of known negative regulators of monocyte/macrophage function. CD 14+ cells were cultured with or without IL-25 in the presence or absence of LPS/PGN for different time points and then examined for expression of SOCS-1 and SOCS-3. LPS and PGN were effective in inducing SOCS-3. This effect was evident as early as 30 minutes after stimulation and was enhanced by IL-25 (Fig. 7A). LPS induced SOCS-1 RNA at 2 hours and this effect was enhanced further by pre-incubation of cells with IL-25 (Fig. 7B). By contrast, induction of SOCS-1 RNA by PGN was seen at 1 hour, but it was not increased by IL-25 (Fig. 7B). These results suggest that SOCS-3 but not SOCS-1 could be involved in the IL-25-mediated negative regulation of inflammatory cytokine by CD14+ cells. The expression of SOCS-3 in CD14+ cells was then eliminated by siRNA. CD14+ cells were stimulated with LPS or PGN in the presence or absence of IL-25. As shown in figure 8, IL-25-mediated inhibition of both TNF-alpha and IL-6 expression following LPS or PGN stimulation was fully abrogated in SOCS-3-deficient cells. Induction of SOCS-3 in several cell types can be mediated by IL-10, an antiinflammatory cytokine that negatively regulates production of inflammatory cytokines by CD14+ cells. Therefore the expression of IL-10 RNA was analysed in cells cultured with or without IL-25 in the presence or absence of bacterial stimuli. However, no significant change in the expression of IL-10 was seen in IL-25-treated cells (Fig. 9). Overall these results indicate that IL-25 is a negative regulator of monocyte/macrophage inflammatory cytokine synthesis.

### IL-25R expression in Inflammatory Bowel Disease (IBD)

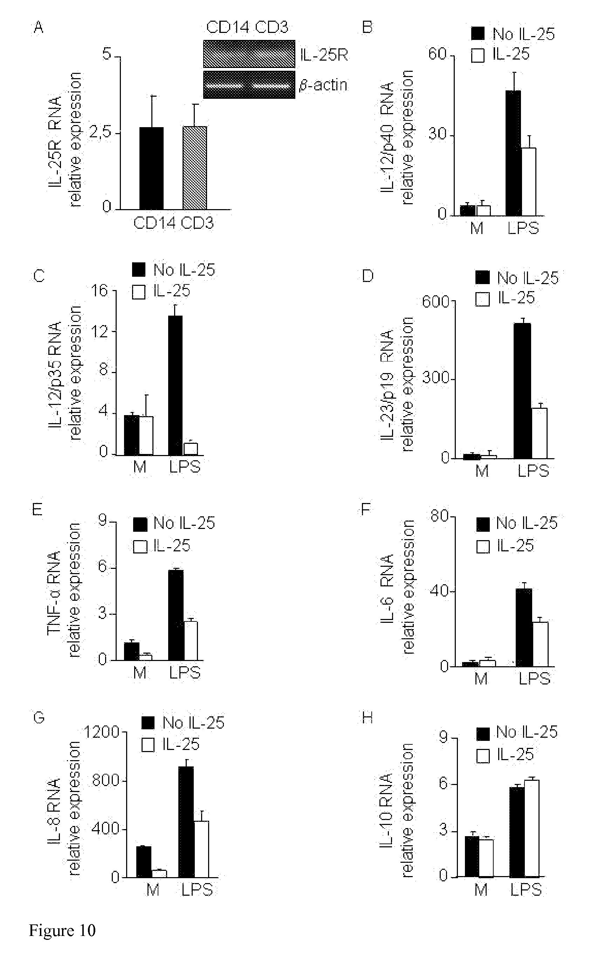

The expression of IL-25R in CD14+ cells isolated from the colon of patients with IBD was analysed to investigate if these CD 14+ cells are functionally capable of responding to IL-25. Real-time PCR analyses showed that IL-25R transcripts were present in mucosal Crohns disease (CD) CD 14+ cells (Fig. 10A). These cells were also shown to express the soluble IL-25R isoform (Fig. 10A, right inset). The treatment of CD CD14+ cells with IL-25 resulted in a significant reduction in the RNA content of inflammatory cytokines induced by LPS (Fig. 10B-G). IL-25 also reduced the constitutive RNA expression of TNF-alpha (Fig. 10E) and IL-8 (Fig. 10 G). In line with data obtained in peripheral blood CD14+ cells, IL-25 did not modify the LPS-induced IL-10 RNA content (Fig. 10H).

### Expression of IL-25 in colon.

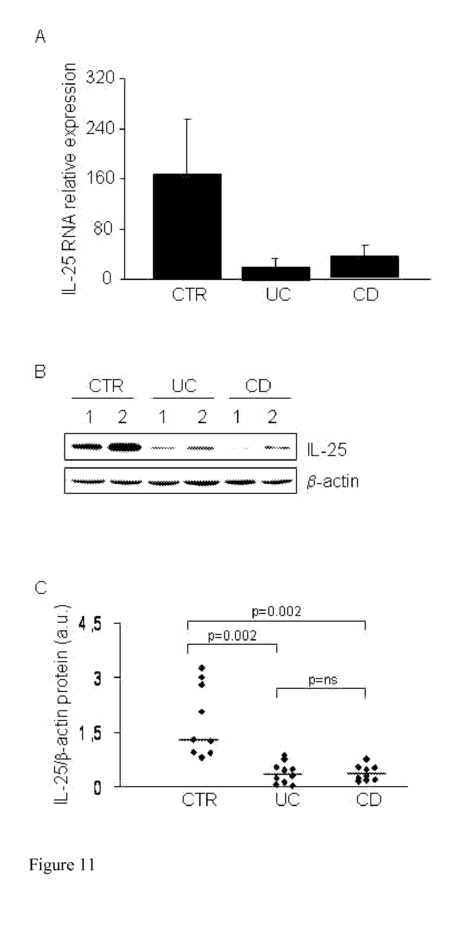

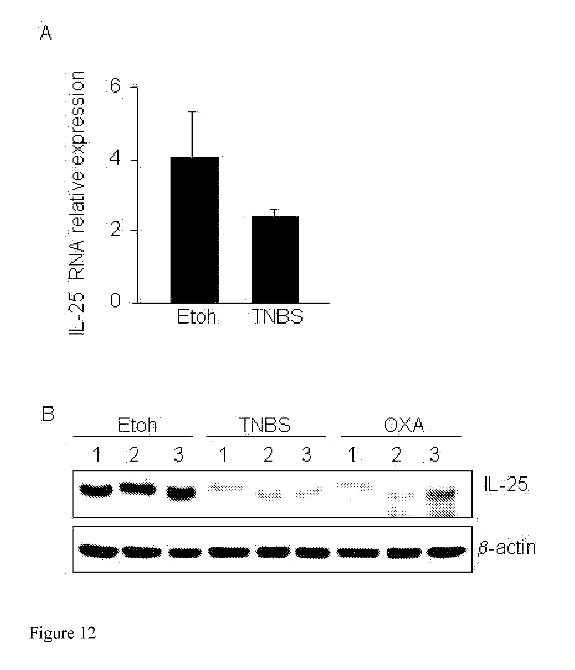

The expression of IL-25 in the human colon was investigated. A constitutive expression of IL-25 RNA and protein was seen in normal colonic samples. Surprisingly, however, a marked down-regulation of IL-25 was seen in tissues from patients with IBD in comparison to the controls, and this was evident at both the RNA (Fig. 11A) and protein level (Fig. 11 B-C). Consistently, IL-25 RNA expression was down-regulated in mice with trinitrobenzene sulfonic acid (TNBS)-colitis, that shows similarities with CD, as compared with ethanol-treated controls (Fig. 12A). Moreover, Western blotting analysis of IL-25 protein content in colonic extracts showed that IL-25 expression is markedly reduced in mice with TNBS or oxazolone-colitis in comparison to controls (Fig. 12B).

### PGN-induced colitis in a mouse model

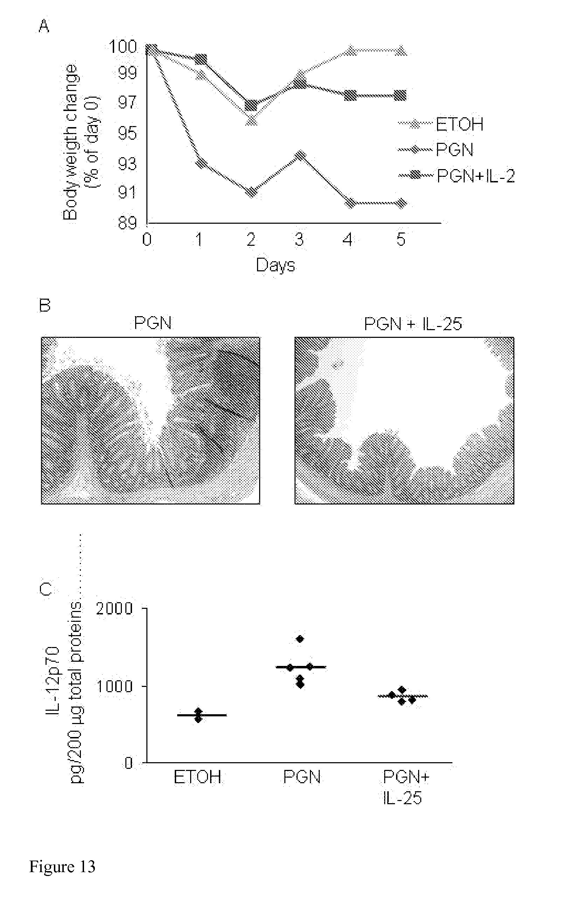

A mouse model of PGN-induced colitis was investigated for response to intraperitoneal injection of IL-25. The colitis was inducible by rectal administration of PGN. As shown in Figure 13(a), intraperitoneal injection of IL-25 prevented the weight loss induced by PGN. Severe mucosal mononuclear cell infiltrate and distortion of the normal crypt architecture with epithelial ulceration and loss of goblet cells was evident in the colon of mice with PGN receiving no IL-25. In contrast, only small scattered areas of cellular infiltration is seen in the colon of mice treated with IL-25 (Figure 13 (b)). Cytokine levels in tissue samples were analyzed by ELISA and data expressed as pg/200 µg total proteins. Each point on the graph illustrated in Figure 13 (c) represents the value of cytokine in colonic samples taken from a single mouse. The horizontal bars indicate the median value.

### IL-25 ameliorates induced PGN-mediated colitis

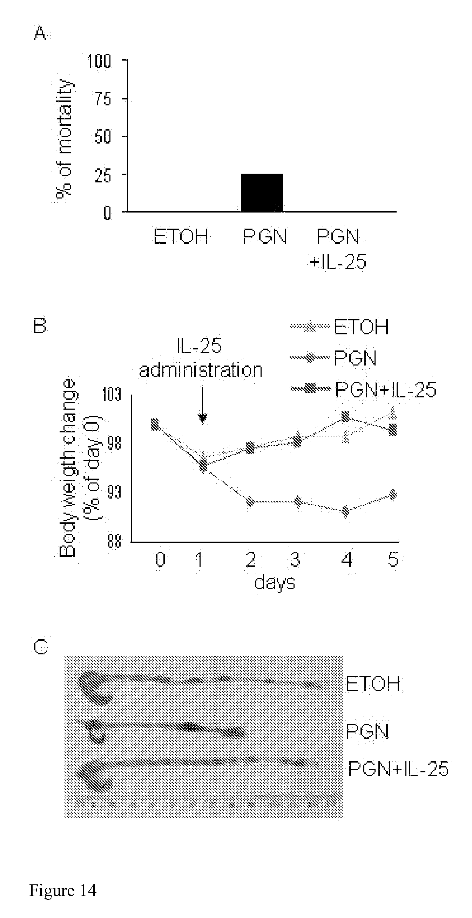

PGN-colitis was induced in Balb/c mice. Control mice were given intrarectal administration of 50% ETOH no PGN. Recombinant mouse IL-25 was administered the day after the induction of colitis. Figure 14 (a) shows a 25% rate of mortality in mice suffering from PGN-mediated colitis left untreated. A 0% rate in mortality was observed in mice suffering from PGN-mediated colitis treated with IL-25 and control mice. IL-25 administration on day 1 resulted in an increase in body weight in mice with PGN-mediated colitis compared to those mice left untreated (Figure 14 (b)) Furthermore, on day 5 there was a significant decrease in body weight in mice suffering with PGN-mediated colitis who were left untreated compared to control mice and those treated with IL-25.

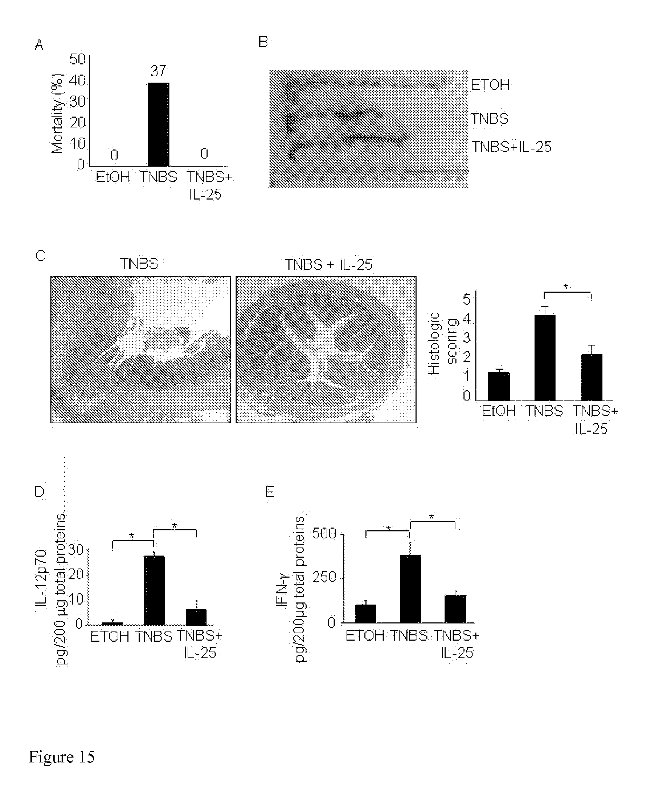

IL-25 was administered to mice with TNBS-induced colitis the day after induction. Mice exhibited a loss of body weight independently of IL-25 treatment (data not shown). A mortality rate of 37% was observed in mice untreated with IL-25. A 0% mortality rate was seen in mice treated with IL-25. Macroscopically, colon samples obtained from mice left untreated appeared shorter than those obtained from mice receiving treatment, as displayed in Figure 15 (b). Consistently, blinded histological scoring of colon tissue was significantly reduced in mice treated with IL-25 compared to mice left untreated (Figure 15 (c)). The effect of IL-25 on Th-1 cytokine production in mice with TNBS-induced colitis was examined. The production of both heterodimeric IL-12/p70 and IFN-γ was significantly increased in the colons of mice with TNBS-colitis in comparison to ethanol treated controls (Figure 15 (d)(e)). Subsequent, administration of IL-25 to mice with TNBS-colitis significantly reduced the production of both of these cytokines.

### Discussion

CD 14+ cells play a central role in regulation of cytokine production observed during the progression of inflammatory diseases such as Inflammatory Bowel Disease (IBD). It is well known in the art that CD 14+ cells are present in the intestinal mucosa and produce inflammatory cytokines, IL-1beta, IL-6, TNF-alpha and IL-8. The molecular mechanisms underlying the enhanced production of inflammatory cytokines by CD14+ cells in IBD are not, however, fully understood. Presently employed methods of treatment for Inflammatory Bowel Disease are plagued with the occurrence of severe side effects and resistance among patients. The present invention provides for an alternative medicament for use in the treatment of Inflammatory Bowel Disease. The present invention relates to a means to regulate the cytokine response of CD 14+ cells.

Interleukin (IL)-25 is a cytokine thought to be involved in the inflammatory response. However, the precise role IL-25 plays in such a response is presently undefined. The current inventors have shown that the receptor responsible for mediating the biological functions of IL-25, namely IL-25R, is present on the surface of CD14+ cells in humans suggesting that CD14+ cells are potential targets of IL-25. The current inventors are the first to show that there is, surprisingly, a defective amount of IL-25 in the inflamed colon of patients with IBD and in experimental mouse models of IBD in comparison to normal control samples.

The current inventors have further shown that IL-25 is a negative regulator of the cytokine response by CD14+ cells in the presence of bacterial and inflammatory stimuli. In addition, IL-25 did not reduce the expression of CD80, CD86 and HLA-DRII, induced by bacterial stimuli in CD14+ cells isolated from the colon of patients with IBD, indicating the IL-25 does not cause a global hypo-responsiveness but rather selectively inhibits the production of inflammatory cytokines. These findings indicate a stimulus-independent inability of CD14+ cells to produce inflammatory cytokines following exposure to IL-25.

The treatment of CD14+ cells isolated from patients suffering from IBD, with IL-25, resulted in a significant reduction in the RNA content of inflammatory cytokines induced by LPS. IL-25 also reduced the constitutive RNA expression of TNF-alpha and IL-8.

The current inventors induced PGN-mediated colitis in a mouse model in order to examine the effect of IL-25 administration. IL-25 administration in mice prevented the induction of PGN-mediated colitis. Histological evaluation of colon samples from mice treated with IL-25, revealed only small, scattered areas of cellular infiltration following administration of PGN. Furthermore, a significant improvement was observed in mice suffering from PGN-mediated colitis following treatment with IL-25 compared to those mice with PGN-mediated colitis left untreated. An additional mouse model of colitis, TNBS-induced colitis, was utilised to evaluate and further confirm the potential therapeutic properties of IL-25. Administration of IL-25 to mice suffering from TNBS-colitis led to a 0% rate of mortality among mice compared to a 37% rate of mortality among mice left untreated. IL-25 treatment also significantly reduced the production of the Th-1 cytokine response in the colon of mice.

The effect of IL-25 on CD14+ cell response was examined for dependency on the induction and/ or activity of known negative regulators of monocyte/macrophage function. These results suggest that SOCS-3 but not SOCS-1 could be involved in the IL-25-mediated negative regulation of inflammatory cytokine by CD14+ cells.

Taken together, the data suggests that a defective IL-25-mediated down-regulation of cytokine response can contribute to amplify monocyte/macrophage cell-mediated immune-inflammatory pathologies. These results indicate that administration of IL-25 could be a potential method of treatment for diseases in which CD14+ plays a part such as Inflammatory Bowel Disease.

## Claims

1. The use of interleukin-25, a derivative of or a fragment of, in the manufacture of a medicament for the treatment of diseases, resulting from cytokine production by CD 14+ cells.

2. A use according to claim 1, wherein the disease is an inflammatory disease.

3. A use according to claim 2, wherein the disease is selected from the group comprising, Inflammatory bowel disease (IBD), Crohn's disease (CD), ulcerative colitis, coronary artery disease, sepsis, arthritis rheumatoid, immune-complex induced and proliferative glomerulonephritis, tubulointerstitial nephritis, vasculitis, viral and autoimmune hepatitis, autoantibody-mediated diseases, psoriasis, neoplasia and other CD 14+ cell related diseases.

4. The use according to any preceding claim involving down regulating the production of inflammatory cytokines.

5. A use according to claim 4, wherein the inflammatory cytokines are involved in a TH1 and/or TH-17 response

6. A use according to claim 5, wherein the inflammatory cytokines are IL-6 and IL-8, TNF-α, IL12, IL-23, IL-22, IL-21 IFN-gamma, IL-17 and IL-1β.

7. A pharmaceutical composition containing a pharmaceutically active amount of interleukin-25, a derivative thereof or a fragment of IL-25, together with a pharmaceutically acceptable carrier or excipent.

8. A method of down regulating the inflammatory cytokine response of CD14+cells comprising administering of interleukin-25, a derivative or a fragment thereof.

9. An antibody targeting IL-25R, for use in the manufacture of a medicament for the treatment of CD14+ associated diseases.

10. The use of interleukin-25, a derivative of or a fragment of, as described herein with reference to the examples.

11. A pharmaceutical composition substantially as described herein with reference to the examples.

12. A method of down regulating the inflammatory cytokine response substantially as described herein with reference to the examples.

13. An antibody substantially as described herein with reference to the examples.
